(19) **Europäisches Patentamt**

**European Patent Office**

**Office européen des brevets**

(11) **EP 1 574 584 B1**

(12) **EUROPEAN PATENT SPECIFICATION**

(45) Date of publication and mention
of the grant of the patent:
**15.11.2006 Bulletin 2006/46**

(51) Int Cl.:
*C12Q 1/04* (2006.01)

(21) Application number: **05004984.0**

(22) Date of filing: **08.03.2005**

(54) **Methods for isolation of bacteria from biological samples**

Verfahren zur Isolierung von Bakterien aus biologischen Proben

Procédé pour isoler des bactéries des échantillons biologiques

(84) Designated Contracting States:
**AT BE BG CH CY CZ DE DK EE ES FI FR GB GR HU IE IS IT LI LT LU MC NL PL PT RO SE SI SK TR**

(30) Priority: **10.03.2004 EP 04005718**

(43) Date of publication of application:
**14.09.2005 Bulletin 2005/37**

(73) Proprietors:
• **Roche Diagnostics GmbH**
**68305 Mannheim (DE)**
Designated Contracting States:
**DE**
• **F. HOFFMANN-LA ROCHE AG**
**4070 Basel (CH)**
Designated Contracting States:
**AT BE BG CH CY CZ DK EE ES FI FR GB GR HU IE IS IT LI LT LU MC NL PL PT RO SE SI SK TR**

(72) Inventors:
• **Klepp, Jürgen, Dr.**
**76676 Graben-Neudorf (DE)**

• **Kaspar, Peter, Dr.**
**82362 Weilheim (DE)**
• **Zielenski, Ralf**
**83671 Benediktbeuern (DE)**
• **Schlipfenbacher, Reiner, Dr.**
**67098 Bad Duerkheim (DE)**

(56) References cited:
**EP-A- 0 605 003**       **WO-A-00/73794**

• **ORLE K A ET AL: "Sensitive and specific detection of sepsis pathogens in 133 muL whole blood using PCR." ABSTRACTS OF THE INTERSCIENCE CONFERENCE ON ANTIMICROBIAL AGENTS AND CHEMOTHERAPY, vol. 43, 2003, page 193, XP001183879 & 43RD ANNUAL INTERSCIENCE CONFERENCE ON ANTIMICROBIAL AGENTS AND CHEMOTHERAPY; CHICAGO, IL, USA; SEPTEMBER 14-17, 2003**

**Description**

This invention is directed to methods for isolation of bacteria from biological samples, especially from blood samples. These methods are suitable for sample preparation of biological samples for nucleic acid-based or immune-diagnostic methods for detection of bacteria. This invention is also related to the use of specific antibodies in methods for isolation of bacteria from biological samples and to kits for conducting these methods.

**Background of the invention**

Determination and isolation of bacteria present in biological samples is a common task within biotechnological applications. For example in medical applications characterization of bacteria present in biological samples derived from man or animal play an important role in diagnosis of infectious diseases. Septicaemia is still a major issue in intensive care with a high mortality rate and tremendous costs for health care system. Today, in most cases blood culture methods are used for diagnosis of sepsis (Weinstein, M.P., et al., Clin. Infect. Dis. 24 (1997) 584-602), which allows specific detection of bacteria within such samples. However, such methods are very time-consuming and very often do not allow to provide the patient with the appropriate therapy in time. Alternative methods allow diagnosis of bacteria by detecting specific proteins and/or nucleic acid sequences of these organisms. Especially, nucleic acid detection methods are becoming increasingly important in view of the progress made in this field during the last years. The nucleic acid amplification methods, especially the polymerase chain reaction allows a very specific, sensitive and fast detection of nucleic acid sequences present in a sample and, therefore, provides an alternative to present culture assays for diagnosis of infectious diseases like sepsis (Martineau, F., et al., J. Clin. Microbiol. 36 (1998) 618-623; Reischl, U., et al., J. Clin. Microbiol. 38 (2000) 2429-2433; Rantakokko-Jalava, K., and Jalava, J., J. Clin. Microbiol. 40 (2002) 4211-4217).
However, such detection methods often require the preparation of a sample prior to the detection of the specific proteins and/or nucleic acids.
It is an object of the present invention to provide improved methods for isolation of bacteria from biological samples. Such methods can be used for sample preparation in diagnostic methods for detecting bacteria in biological samples, especially in blood samples.

Summary of the invention

The main object of the invention is to provide methods for isolation of bacteria from a biological sample using antibodies specific for eukaryotic cells contained in said sample, whereby said antibodies are deficient of a bacteria-binding Fc-terminus.
A preferred embodiment of the present invention is a method for isolation of bacteria from a biological sample comprising the steps:

- providing antibodies specifically binding to eukaryotic cells contained in said biological sample, whereby said antibodies are deficient of a bacteria-binding Fc-terminus, and
- mixing said antibodies with said biological sample, and
- separating the antibody-eukaryotic cell-complexes from said mixture.

It is important to use antibodies deficient of bacteria-binding Fc-termini in such methods, at least these antibodies do not bind to those bacteria which should be detected subsequently. The Fc-terminus of antibodies commonly used in biotechnological applications most often are capable of binding to nearly all bacteria over immunoglobulin binding proteins like protein A, protein G and protein L (Navarre, W.W., and Schneewind, O., Microbiol. Mol. Biol. Rev. 63 (1999) 174-229; Reeves, H.C., et al., Anal. Biochem. 115 (1981) 194-196); Nilson, B., et al., J. Immunol. Methods 99 (1987) 39-45); Åkerström, B., et al., J. Biol. Chem. 264 (1989) 19740-19746). However, using such antibodies in an isolation method as described above not only lead to depletion of eucaryotic cells, but also to depletion of bacteria in the samples. This necessarily would lead to an underestimation of the bacterial load in the sample or in the worst case to false negative results in subsequent methods for detecting bacterial nucleic acids or proteins. Therefore, it is very important to use antibodies deficient of bacteria-binding Fc-termini. Especially suitable for this purpose are tetrameric antibodies (US 2003/0092078), Fab-fragments and antibodies having a masked Fc-terminus.
Another embodiment of this invention is directed to the extraction of nucleic acid and/or proteins from the biological sample following depletion of eukaryotic cells from said sample. The extracted protein and/or nucleic acids derived from said sample subsequently can be used in methods for detecting bacteria-specific proteins and/or nucleic acids.

**Detailed description of the invention**

One embodiment of the present invention is directed to methods for isolation of bacteria from biological samples by depleting eucaryotic cells present in said samples.

Samples depleted from eucaryotic cells do have some advantageous properties, for example, when detecting bacteria using immunodiagnostic- or nucleic acid detection methods. Namely, the level of eucaryotic proteins and nucleic acids normally present in biological sample, especially in blood samples, compared to the level of bacterial nucleic acids and proteins, is very high. This could disturb detecting bacterial nucleic acids and/or proteins in such samples. This is of an especial importance when extracting total nucleic acids and/or proteins from these samples prior to detecting specific nucleic acids and/or proteins.

This can be exemplified when detecting bacterial nucleic acids in such samples using the PCR method. PCR allows amplification and detection of theoretical one target present in a sample (however, in practice, this sensitivity is very difficult to achieve). Beside primer and probe optimization, the sensitivity of a PCR-assay is strongly influenced by the ratio of target DNA to background DNA. It is well known that with an increasing amount of background DNA the sensitivity of a PCR assay for the target DNA may be diminished.

In samples from man or animal most of the nucleic acids are derived from eucaryotic blood cells present in these samples and not from bacteria to be detected. The ratio of bacterial nucleic acids compared to human nucleic acids can be easily calculated. 1 ml of whole blood from a healthy human donor contains between $3 \times 10^6$ and $10 \times 10^6$ leukocytes. In case of sepsis patients, leukocyte levels are elevated up to $30 \times 10^6$/ml. The assumption can be made that a "typical" sepsis patient has a leukocyte content of $10 \times 10^6$/ml and a bacterial load of 100/ml. Taking into account that the size of the human genome is in the range of $3 \times 10^9$ base pairs haploid ($6 \times 10^9$ base pairs diploid) per leukocyte and the size of the bacterial genome is in the range of $6 \times 10^6$ base pairs, this results in a ratio of bacterial target DNA to human background DNA of 1 to $10^8$.

A common practice to overcome the problem of inhibition of background nucleic acids is to use internal controls during amplification and to dilute inhibited samples in subsequent PCR runs. However dilution of samples normally leads to a loss in sensitivity, which should be avoided. And also additional dilution steps and PCR amplification reactions are not preferred in routine diagnostic methods. When aiming to detect bacteria in a typical blood sample derived from a patient, it would therefore be favorable to overcome the problem, that most of the total nucleic acids extracted from these samples are derived from the donor. In addition, especially with regard to samples from sepsis patients it is not possible to get high volume samples which could circumvent sensitivity problems in nucleic acid detection methods.

The present invention provides a solution to this problem by allowing selective depletion of eukaryotic cells from the sample. This sample does not contain high concentrations of donor nucleic acids and can be used to prepare nucleic acids from the pathogenic agent, especially from the bacteria contained in the sample.

Although this especially exemplifies the problem when detecting nucleic acids it should be noted that there are similar problems for detection of bacterial proteins. In such methods proteins from the donor can lead to significant disturbance. In addition such methods can also be used to improve methods for detecting other pathogens such as viruses.

The biological sample can be derived from human, animal or elsewhere in nature. Preferred samples are blood, serum, plasma, bone marrow, tissue, sputum, pleural and peritoneal effusions and suspensions, urine, sperm and stool.

Bacteria in the context of the present invention can refer to any bacteria known, especially to bacteria which are involved in pathogenic conditions, for example, in infectious diseases.

Of special interest are bacteria involved in sepsis, like Staphylococcus spp, Streptococcus spp, Enterococcus spp, Enterobacter spp, Klebsiella spp, Escherichia coli, Proteus mirabilis, Pseudomonas spp, Haemophilus influenzae and others. The present invention allows detection of several bacteria involved in such diseases by conducting only one isolation method depleting eucaryotic cells from that sample, extracting proteins and/or nucleic acids and subsequently detecting nucleic acids and/or proteins specific for one or more bacteria involved. Such multiplex detection methods are difficult to conduct using sample preparation methods known in the art.

Most often it is not necessary to deplete all eukaryotic cells present in a biological sample in order to achieve the desired effect. For immune-diagnostic methods for example it may be sufficient to deplete certain eukaryotic cells having a greater cross-reactivity when using a certain antibody or to decrease the content of eukaryotic proteins by depleting the most abundant cells. For nucleic acid detection methods, it is in most cases sufficient to deplete nucleated eukaryotic cells, which do have genomic DNA. Depletion of erythrocytes is in most cases not necessary, as these cells do not have genomic DNA and inhibitors contained in these cells can easily be washed away during the subsequent sample preparation method. Also, especially when conducting nucleic acid amplification methods, it is foremost desired to increase significantly the relative content of bacterial nucleic acids over eukaryotic genomic DNA. Therefore, it is sufficient to deplete most of these cells, but it is not necessary that the depleted biological sample is free of all eukaryotic cells.

The antibodies used in the method of the present invention have to fulfill two essential properties. Firstly, they do bind to eukaryotic cells which should be depleted from a biological sample, preferably by the specific antigen-binding domains of these antibodies. For example, for blood samples, suitable antibodies specifically binding to cell surface antigens of leucocytes, erythrocytes, monocytes are known to people skilled in the art. (e.g. CD2/CD3 for T cells, CD14 for Monocytes, CD15 for Granulocytes and Monocytes, CD16 for Macrophages, CD36 for Platelets, Monocytes and Macrophages, CD45 for Leucocytes).

Secondly, the antibodies are deficient of a bacteria-binding Fc-terminus or the Fc-terminus of the antibody is blocked (e.g. when using tetrameric antibodies). Antibodies with Fc-termini which bind bacteria, would result in eukaryotic cell-antibody complexes also containing bacteria. Separation of the complexes from the sample would unintentionally lead to a sample which is also depleted from the bacteria. This would lead to false-negative results in subsequent bacteria detection methods conducted on the sample. Especially, the Fc-termini of IgG-antibodies, which are commonly used in biotechnological methods, bind bacteria with a high affinity.

Antibodies deficient of binding bacteria to the Fc-termini, which can be used in methods of the present invention, are for example tetrameric antibodies or antibody-fragments lacking the Fc-part like Fab- or F(ab')$_2$-fragments generated by Papain or Pepsin digestion, which is a state of the art procedure for skilled people.

However, if only a special species of microorganisms shall be detected from the biological sample, IgM-type antibodies can be used for the depletion of the eukaryotic cells from the sample, as some microorganisms like Staphylococcus aureus or Streptococcus spp express only immunoglobulin-binding proteins like Protein A or Protein G showing a strong binding to the Fcγ-part of IgG's but (nearly) no binding to IgM, whereas other microorganism like Peptostreptococcus magnus express Protein L, which strongly binds both IgG and IgM.

Therefore, the use of IgM-type-antibodies is an alternative embodiment of the present invention as this approach would be not universal but limited to the detection of special microorganisms, like Staphylococcus aureus and Streptococcus spp.

Depending on the properties of the antibodies, the antibody-eucaryotic cell-complexes can be separated from the biological sample by standard methods known in the art.

Such complexes can for example be separated from the sample by using matrixes capable of binding the antibodies. If the complexes are different in their bouyant density compared to the bacteria, the complexes can easily be separated for example by density gradient centrifugation of the sample. When using cross-linked antibodies, like IgM or tetrameric antibodies, the complexes are very dense and can be very easily pelleted by a one step density gradient using e.g. Fc cell ($\rho$ ~ 1,080 g/ml) centrifugation. Another possibility is to use antibodies coupled directly or indirectly to a solid phase, like for example magnetic particles. The antibody-eucaryotic cell-complexes can then be separated very easily by applying magnetic force. When directly linked to the solid phase the antibodies are coupled over a covalent bond to the solid phase using techniques known in the art. Indirect linkages are also known in the art, for example streptavidin - biotin and antibody - antigen- pairs (as digoxygenin - anti-Digoxygenin antibody).

In a further embodiment of the present invention, bacteria were not only isolated from biological samples by using antibodies specific for eukaryotic cells, which are deficient of a bacteria-binding Fc-terminus for depletion of eukaryotic cells from said biological samples, but further extracting nucleic acids and/or proteins from said processed samples. For this purpose, standard extracting methods known in the art can be used (see for example Sambrook et al., Molecular Cloning - A Laboratory Manual, Cold Spring Harbor Laboratory Press (1989)).

Nucleic acids for example can be prepared by lysing these cells, digestion with proteinase K, optionally conducting a phenol/chloroform extraction and precipitating the nucleic acids using acetone or propanol as commonly known in the art (Sambrook et al., supra). However, also many alternative methods can be used, like easy-to-use extraction kits, commercially available based for example on the glass-nucleic acid binding-technique (for example MagNAPure® sold by Roche Diagnostics).

A further embodiment of the present invention is directed to the isolation of bacteria from biological samples by depleting eukaryotic cells using eukaryotic cell-specific antibodies, which are deficient of a bacteria-binding Fc-terminus, extraction of nucleic acids and/or proteins from said samples and detecting specific bacteria nucleic acid sequences and/or proteins in said sample. Suitable detection methods are not limited to distinct methods known in the art (see for example Sambrook et al., supra).

Bacteria-specific nucleic acid sequences can be detected by methods known to an expert, for example by probe-hybridization methods using Southern Blot techniques. Other detection methods include sequencing of the nucleic acid sequences to be detected or cloning of the desired nucleic acid sequences in plasmid vectors. An overview is given in (Sambrook et al., supra).

If the target nucleic acid is only present in very low concentrations in the sample, amplification methods are useful in order to allow detection. Suitable amplification methods are for example LCR (U.S. Patent Nos. 5,185,243, 5,679,524 and 5,573,907; EP 0 320 308 Bl; WO 90/01069; WO 89/12696; and WO 89/09835), cycling probe technology (U.S. Patent Nos. 5,011,769, 5,403,711, 5,660,988, and 4,876,187, and PCT published applications WO 95/05480, and WO 95/00667), Invader TM technology (U.S. Patent Nos. 5,846,717; 5,614, 402; 5,719,028; 5,541,311; and 5,843,669), Q-Beta replicase technology (U.S. Patent No. 4,786,600) , NASBA (U.S. Patent No. 5,409,818; EP-0 329 822), TMA (U.S. Patent Nos. 5,399,491, 5,888,779, 5,705,365, 5,710,029), SDA (U.S. Patent Nos. 5, 455,166 and 5,130,238) and PCR (US-A-4,683,202).

The invention furthermore refers to kits, which can be used in the methods described above.

Preferred kits for extracting bacterial nucleic acids and/or bacterial proteins from a biological sample comprising:

- in one or several containers antibodies specifically binding to eukaryotic cells in that biological sample, whereby that antibodies are deficient of a bacteria-binding Fc-terminus,

- in one or several containers means for extracting nucleic acids and/or proteins.

Means for extracting nucleic acids and/or proteins are reagents or devices for extracting nucleic acids or proteins, like Proteinase K, (Nucleic acid) binding buffer, (Nucleic acid) washing buffer, (Nucleic acid) elution buffer and, if needed, also other reagent can be contained in these kits.

A further embodiment of the present invention is directed to kits also containing means for detecting nucleic acids and/or proteins. Such kits can also be used for detection. Detection means can be for example an antibody specific for bacterial proteins. If bacterial nucleic acids should be the target, suitable means are bacteria-specific oligonucleotide probes and suitable hybridization buffers. In case the target nucleic acid should be amplified, also amplification means could be contained in these kits, for example primer(s), amplification buffers, probes and/or amplification enzymes.

Means for detection, like antibodies, oligonucleotides, such as primers and probes, could be optionally labelled in order to simplify detection. Suitable labels are known in the art.

The present invention is exemplified by the following examples:

## Examples

### Example 1

**Isolation of bacteria from blood samples by depletion of leukocytes using density gradient centrifugation**

**Background of the approach**

The use of density gradient media is a common way in clinical chemistry to separate blood cells into different populations by centrifugation. The most popular media used are Percoll® and Ficoll®. Percoll® is a polydisperse colloidal silica sol in the range of 15 to 30 nm, coated with nondialyzable polyvinylpyrrolidone (PVP). Commercial available Percoll® (e.g. from Amersham) consists of about 23 % (weight per weight) of silica particles giving a density of 1.130 $\pm$ 0.005 g/ml. Ficoll-Paque Plus® from Amersham is an aqueous solution of 5.7 g Ficoll® 400 (a synthetic high molecular weight polymer of sucrose and epichlorhydrin) and 9.0 g sodium diatrizoate per 100 ml, giving a density of 1.077 $\pm$ 0.001 g/ml.

In principle two techniques are used for cell separation: continuous and discontinuous (step-wise) density gradients. In case of a continuous gradient, a suspension of particles (e.g. cells) is centrifuged and the cells sediment to the position of the gradient, where the density of the cells and the density of the gradient is equivalent (buoyant density of the cells). Cells differing in density in as little as 0.01 g/ml can be separated by this technique. When using discontinious gradients, cells sediment to the interface of two different dense media, where the upper media has a lower and the lower media has a higher density than the sedimented cells.

The sedimentation rate v (which is a velocity) of a particle is given by stokes law

$$V = \frac{d^2(\rho p - \rho i)}{18\eta} \times g$$

which means

- that the sedimentation rate increases as the centrifugal force (g) is increased.
- that the sedimentation rate is proportional to the square of the particle size (d).
- that the sedimentation rate is proportional to the difference between the density of the particle ($\rho p$) and that of the surrounding media ($\rho i$), which means that the sedimentation rate becomes zero when the density of the particle and the density of the media are equivalent.
- that the sedimentation rate decreases as the viscosity of the media ($\eta$) increases.

As the formation of continuous Percoll® gradients is time consuming and high g-forces are needed (20.000 — 35.000 g), the experiments described below were performed with discontinuous one or two step gradients, where the density of the media is given by diluting the Percoll with isotonic NaCl-solution.

In this case, the density steps in the centrifuge tube are simply made by pipetting and overlaying one media after another, with the whole blood sample having the lowest density on top of the tube.

The following table shows the buoyant densities of different blood cells and *E. coli,* taken from a technical application note from Amersham/Pharmacia for the use of Percoll®.

**Table 1**:

|  | Density (g/ml) |
|---|---|
| Human blood cells |  |
| Thrombocytes | 1.04 - 1.06 |
| Lymphocytes | 1.06 - 1.08 |
| Granulocytes | 1.08 - 1.09 |
| Erythrocytes | 1.09 - 1.10 |
|  |  |
| *E. coli* | 1.13 |

Due to this list, the assumption was that bacteria have a density which is distinct higher than the density of white blood cells and therefore routine protocols able to separate lymphocytes and monocytes (PBMCs) from granulocytes and from erythrocytes should be adaptable to separate intact bacteria from white blood cells.

**Experimental set-up**

In a 15 ml Falcon tube a two step Percoll® gradient was made by first pipetting 4 ml of a 74 %ig isotonic Percoll® solution ($\rho \sim 1.095$ g/ml) into the tube, overlaying this media with 4 ml of a 55 %ig isotonic Percoll solution® ($\rho \sim 1.075$ g/ml) and overlaying both density media with 4 ml of bacteria spiked whole blood.

This two step gradient containing the sample was centrifuged for 20 minutes at 350 g at room temperature in a Heraeus Variofuge 3.0 R with a swing out rotor (type 05315) and the amount of blood cells in the fractions and/or in the cellular interfaces formed between the media was determined by measuring aliquots of these fractions on a Beckman Coulter AcT Diff.

The amount of human genomic DNA in the fractions was determined by amplifying the β-Globin gene on a LightCycler® 1.2 using the LightCycler-Control Kit® DNA, the amount of bacterial DNA *(Staph. aureus* and P. *aeruginosa)* by using single parameter assays from Roche Diagnostics.

For this purpose, aliquots of the fractions were processed on the MagNA-Pure® following the instructions given in the manual.

Recovery of human genomic DNA and bacterial DNA in the fractions was calculated by processing an "untreated" blood sample aliquot on the MagNA Pure and setting the concentration of this not centrifuged sample as 100 %.

Volume ratios between cellular fractions and initial sample volume were taken into account when calculating the recoveries of the centrifuged samples.

Modifications of this protocol like variation of g-forces, centrifugation time and changes in the density of the media are discussed below.

**Results and discussion**

Using the two step gradient as described above (20 minutes 350 g), a whole blood samples is separated nearly quantitatively into 3 fractions.

The first fraction is a compact white cellular layer located at the interface between the "plasma" and the 55 %ig Percoll, consisting of concentrated platelets and peripheral blood mononuclear cells (PBMC = lymphocytes and monocytes).

The second fraction consists of concentrated granulocytes (polymorph nuclear cells), located at the interphase between the 55 %ig and the 74 %ig Percoll® and the third fraction is a red pellet of erythrocytes at the bottom of the tube, as the red blood cells have a slightly higher density than the 74 %ig Percoll® solution. (In some cases, the erythrocytes gave a cloudy pellet distributed about the whole volume of the 74 %ig Percoll® fraction, which was caused by samples having low amounts of hemoglobin per erythrocytes and therefore a lower buoyant density.)

Assuming a higher buoyant density of the bacteria compared to blood cells, the bacteria should sediment together with the erythrocytes to the bottom of the tube, therefore being separated from the white blood cells.

As bacteria are in the range of about 1 $\mu$m whereas blood cells are in the range of about 10 $\mu$m and as the sedimentation rate v is a function of the square of the cell diameter ($d^2$), bacteria should sediment extremely slow compared to blood cells at moderate g-forces.

Model calculations according to the stocks equation gave sedimentation times of about 6 hours for the above-described centrifugation conditions to concentrate the bacteria at the bottom of the tube, which is not only caused by the small particle size but likewise by the low difference in density between the 74 %ig Percoll® and the bacteria.

Therefore in a further set of experiments the protocol was adapted to higher g-forces resulting in higher sedimentation rates/velocities, which is limited by the phenomena, that at too high g-forces the silica particles of the Percoll® begin to sediment (forming a continious gradient) and the system becomes "instable".

Centrifugation for up to 2 hours at 2300 g was possible without destroying the steps of the density gradient, still separating the blood cells into the 3 fractions described above.

Furthermore the two step gradient was simplified to a one step gradient containing only 4 ml of whole blood and 4 ml of 74 %ig Percoll®. In this case the cellular fraction at the interface plasma/Percoll® contained all subpopulations of the white blood cells (and the thrombocytes) and the red blood cells were pelleted at the bottom of the tube.

The advantage of this one step gradient is that the distance of the bacteria to sediment to the bottom of the tube is distinct shorter, and therefore the bacteria should sediment (combined with the higher g-forces) to the bottom of the tube in about 1 hour.

With this optimized protocol, bacteria spiked whole blood from 10 different donors was centrifuged and analysed.

The supernatant including the cellular fraction at the interface plasma/Percoll® contained about 90 % of the human genomic DNA, which was in accordance with the corresponding amount of leukocytes found by the Coulter Counter.

Surprisingly about 80 % of the bacteria were likewise found in this fraction and not as expected in the 74 %ig Percoll® phase (see table below), which means that it is not possible to separate the white blood cells and the bacteria into the two different phases.

Furthermore there was nearly no difference between a "soft-spin" (30 minutes at 350 g) and a "hard spin" (100 minutes at 2300 g), which indicates that the buoyant density of the bacteria must be lower than the density of the 74 %ig Percoll® solution ($\rho$ = 1.095 g/ml).

Therefore in a last set of experiments, the density of the Percoll® solution was decreased using 65 % and 55 % Percoll® to enable the bacteria to penetrate, together with the erythrocytes, into the Percoll® fraction.

In this case the granulocytes, which are the most dense white blood cells, already went into the Percoll® phase, whereas about 70 % of the bacteria and all lymphocytes and monocytes still stayed at the interphase/in the supernatant.

This means that the density of the Percoll® is still higher than the buoyant density of most of the bacterial cells.

**Table 2:**

| Recovery of DNA (at 100 minutes 2300 g) | 74 % Percoll $\rho$ = 1.095 | 65 %Percoll $\rho$ = 1.085 | 55 % Percoll $\rho$ = 1.075 |
|---|---|---|---|
| Supernatant/ interphase | ~ 90 % h.gen. DNA<br>~ 80 % bacterial DNA | ~ 40 % h.gen. DNA[∃]<br>~ 70 % bacterial DNA | ~ 40 % h.gen. DNA[∃]<br>~ 70 % bacterial DNA |
| Percoll phase | ~ 10 % h.gen. DNA<br>~ 20 % bacterial DNA | ~ 60 % h.gen. DNA[#]<br>~ 30 % bacterial DNA | - 60 % h.gen. DNA[#]<br>- 30 % bacterial DNA |
| [∃]According to Coulter Counter mainly lymphocytes and monocytes | | | |
| [#]According to Coulter Counter mainly granulocytes | | | |

As these results are in clear contradiction to the initial assumption that bacteria have a buoyant density greater than 1.10 g/ml (as stated in the technical note of Amersham/Pharmacia for the use of Percoll®), an own literature search was made.

Bakken, L.R., and Olsen, R.A. (Appl. Environ. Microbiol. 45 (1983) 1188-1195) published values between 1.035 g/ml and 1.093 g/ml for the buoyant densities of several bacteria. But even for one species (*E. coli)* values for the buoyant density are varying between 1.05 g/ml and 1.10 g/ml (see e.g. Woldringh, C.L., et al., J. Bacteriol. 148 (1981) 58-63).

The difference in the values reported is partly caused by different techniques/media used, causing different osmotic effects and salt penetration into the cells thereby influencing the buoyant density of the cells.

Furthermore literature is stating that growth conditions will influence the buoyant density of bacterial cells (see e.g. Martinez-Salas, E., et al., J. Bacteriol. 147 (1981) 97-100).

**Conclusions**

The use of density gradient media is a common way in clinical chemistry to separate blood cells into different populations by centrifugation. Due to the relatively dense hemoglobin molecules in red blood cells, the erythrocytes sediment to the bottom of the tube during the centrifugation. As white blood cells are, from a morphological point of view, heterogenious classes of cells, the buoyant density of these cells range from 1.06 g/ml for mononuclear cells (lymphocytes and monocytes) up to 1.09 g/ml for polymorph nuclear cells (granulocytes).

As a consequence white blood cells can be separated into different fractions depending on the density of the media used for centrifugation.

Bakken and Olsen (1983, see above) published values between 1.035 and 1.093 g/ml for the buoyant density of several bacteria using Percoll®.

According to the above results, the buoyant density of bacterial cells (experiments with *Staph. aureus* and P. *aeruginosa)* seems to be in the same range as the buoyant density of mononuclear white blood cells (~ 1.06 - 1.07 g/ml). As a consequence, the separation of all white blood cells and bacteria into two different dense media appears to be not possible.

Using 74 % Percoll® ($\rho$ ~ 1.095 g/ml) only the erythrocytes penetrate into the Percoll® and the bacteria stay together with the lymphocytes, monocytes and granulocytes in the supernatant.

When decreasing the density of the Percoll® to $\leq$ 1.085 (= $\leq$ 65 %) the granulocytes sediment together with the erythrocytes to the bottom of the tube, where-as the bacteria still stay, together with the mononuclear white blood cells, in the supernatant.

Therefore an approach, where the "less dense" white blood cells are co-precipitated to the "more dense" erythrocytes, followed by a centrifugation step using a media with a density higher than the buoyant density of the bacteria should result in a separation of the bacterial cells from all white blood cells. This approach will be described in Example 3.

## Example 2

**Isolation of bacteria from blood samples using Dynal® beads**

**Background of the approach**

Depletion of leukocytes (and subpopulations of them) by immunocapturing is an established way to enrich rare cells (e.g. tumor cells) from blood samples. As different types of white blood cells express different types of CD-surface antigens, mixtures of magnet-beads are used, and the leukocytes are depleted by magnet-separation.

**Experimental set-up**

1 ml of whole blood was incubated for 20 minutes at room temperature with 70 $\mu$l of Dynabeads® M-450<CD45> (Dynal Prod. No. 111.19) and/or 70 $\mu$l of Dynabeads M-450<CD15> (Dynal Prod. No. 111.17) on a rolling incubator. As lymphocytes mainly express CD45 on the cell surface, whereas monocytes and granulocytes mainly express CD15, a mixture of both magnet- beads is necessary to reach an acceptable depletion rate for all white blood cells. After magnet separation of the beads, the depletion rate in the supernatant was determined by measuring the remaining blood cells on a Beckman Coulter AcT Diff. The supernatant was then digested by lytic enzymes or by bead beating on a Ribolyzer using "blue beads" and the sample was processed on the MagNA Pure® according to the protocol described in the manual/package insert.

The amount of human genomic DNA in the eluate was quantified by amplifying the $\beta$-Globin gene on a LightCycler 1.2 (Roche Diagnostics) using the LightCycler-Control Kit DNA (Roche Cat. No. 2 158 833), the amount of bacterial DNA by using single parameter assays for *Staph. aureus* and *P. aeruginosa.*

**Results and discussion**

Using a mixture of <CD45> and <CD15> beads as described above, the depletion rate for leukocytes and the corresponding human genomic DNA was up to 90 %.

The following table shows the recovery of a gram positive and a gram negative bacterium in the supernatant of spiked whole blood (100 bacteria/PCR) after immunocapturing of the leukocytes.

Recovery of bacterial DNA was calculated by processing an untreated blood sample aliquot on the MagNA Pure® and setting the concentration of the sample as 100%.

**Table 3:**

| Beads | % recovery of | | Depletion rate of leukocytes in % |
|---|---|---|---|
| | *Staph. aureus* | *P. aeruginosa* | |
| <CD45> | 34 % | 38 % | 73 % |
| <CD15> | 114 % | 85 % | 57 % |
| <CD45> + <CD15> | 49 % | 42 % | 89 % |

The recovery rate for the bacteria is in the range of 100 % when incubating the spiked samples only with <CD15> beads. Using the same amount of <CD45> beads or adding the <CD45> beads to the <CD15> beads decreases the recovery of the bacteria in the range of about 40 %, which means that beside the leukocytes the majority of bacteria bind to the <CD45> beads.

It was shown that the binding of the bacteria to the <CD45> beads is not white blood cells mediated by repeating the experiment with bacteria spiked plasma as sample material.

Furthermore it is unlikely that the bacteria bind unspecifically to the <CD45> IgG-coated beads, as the addition of different surfactants (NP-40, Na-Laurylsarcosin, Zwittergent 3-12®) to the blood sample in a concentration range where the leukocytes are not yet lysed (0.05 % to 0.5 %) does not reduce the undesired binding of the bacteria to the beads.

The most probable explanation is that the bacteria bind via Immunglobulin Binding Proteins to the $F_{c\gamma}$-part of the IgG coated on the <CD45> beads. *Staphylococcus aureus* for example expresses Protein A as an Immunoglobulin Binding Protein on the cell surface.

This would explain why nearly no binding of the bacteria to the <CD15> beads occurs, as the <CD15>Ab on these beads is an IgM and Protein A has no affinity to IgMs.

**Conclusions**

Depletion of leukocytes via <CD45>/<CD15> magnet beads is an established tool in cell separation (e.g. enrichment of tumor cells).

It was found that bacteria bind to the <CD45> beads, probably via Immunoglobulin binding proteins expressed on the cell surface of the bacteria to the mouse-IgG-antibody coated on the surface of the beads. When using <CD15> beads, which contain a mouse-IgM-antibody, no binding of the bacteria to the beads was found.

**Example 3**

**Isolation of bacteria from blood samples by depleting leukocytes using tetrameric antibodies and centrifugation**

**Background of the approach**

The company Stemcell (Vancouver Canada) offers in there RosetteSep® product line several antibody cocktails for the depletion of blood cells. These RosetteSep® reagents crosslink unwanted cells (e.g. leukocytes) to multiple red blood cells, forming rosettes. When centrifuged over an buoyant density media like Ficoll® ($\rho \sim 1.080$ g/ml), the unwanted (rosetted) cells pellet along with the free RBCs ($\rho \sim 1.09 - 1.10$ g/ml), leaving the desired cells (e.g. tumor cells) untouched, staying in the plasma supernatant or, depending on the centrifugation conditions, at the Ficoll/plasma interphase.

The tetrameric antibody complexes of the cocktail consist of two mouse-IgG-antibodies, one directed against surface antigens of the leukocytes (CDxx), the otherone directed against glycophorin A as a surface antigen expressed on erythrocytes and two <MouseFcγ>Rat-IgM-antibodies bridging the two mouse antibodies via the Fcγ-part to a tetrameric complex.

These reagents are routinely used for tumor cell enrichment, giving (according to the manufacturer) a depletion rate for the rosetted cells in the range of 2 to 3 orders of magnitude by a recovery rate of the tumor cells of about 30 %.

Concerning the immunprecipitation step, no significant unspecific binding of the bacteria to the antibodies of the cocktail was expected (as it was seen for the use of Dynabeads M 450 <CD45>, see Example 2), because in this approach, the Fcγ-part of the mouse IgG's used is hidden by the bridging rat-IgM antibodies and immunoglobulin binding proteins expressed by bacteria show no or only a very weak interaction with rat-IgM.

In earlier experiments using density gradient media (see Example 1) it was observed, that bacteria can not penetrate into density media having buoynat densities $\geq 1.070$ (55 - 74 % Percoll).

Therefore it is expected, that bacteria should stay in the supernatant during the soft spin, whereas the relatively dense erythrocytes and the leuko-coprecipitates would be separated by forming a pellet in the Ficoll- phase.

**Experimental set-up**

The starting point for the experiments with bacteria spiked blood samples was a protocol taken from a technical application note from Stemcell for the depletion of leukocytes.

The protocol uses the antibody cocktail called "CD45 Depletion for Enrichment of Circulating Epithelial Tumor Cells", Cat. No. 15 122 (2 ml for labeling 40 ml of whole blood) which is directed, beside <CD45>, against <CD66b> and <CD36>.

Furthermore a special density medium called DM-L (Cat. No. 15 705, 100 ml; $\rho$ = 1.081 g/ml) is used. Stemcell states that the commonly used Ficoll ($\rho$ = 1.077 g/ml) can likewise be used giving a little lower recovery rate for the tumor cells in the supernatant.

According to this protocol, 2.0 ml of whole blood were incubated with 100 $\mu$l CD45 depletion cocktail for 20 minutes at room temperature by gentle shaking in an Eppendorf mixer. The sample was diluted with 2.0 ml PBS containing 2 % RPLA-4 (bovine plasma albumin). 3.0 ml of DM-L density media was pipetted into a 15 ml conical bottom Sarstedt tube (Cat. No. 62.554.502 PP) and the diluted sample was layered over the Ficoll-like media. The sample was centrifuged for 20 minutes in a Heraeus Variofuge 3.0 R using a swing out rotor (type 05315) at 2700 rpm (= 1200 g).

After centrifugation, the interphase between the generated "plasma" and the Ficoll-like media containing the pelleted blood cells was clearly visible. The two phases were separated by pipetting and aliquots of them were measured on the Beckman Coulter Counter and compared to the initial cell count of the sample to determine the depletion ratio for the leukocytes.

The amount of human genomic DNA and bacterial DNA was determined by processing 750 $\mu$l aliquots of both phases on the MagNA Pure from Roche Diagnostics according to the protocol described in the manual/package insert. The DNA in the eluates was quantified by LightCycler®-PCR as described earlier and expressed as recovery rates for bacteria and depletion rates for human genomic DNA taking the DNA content of the MagNA Pure processed samples without a previous immunprecipitation step as the 100 % value.

**Results and discussion**

Using the original Stemcell protocol as described above, no leukocytes and erythrocytes were detected by cell count in the plasma phase after centrifugation. Even the Ficoll-like phase was, beside a compact cell pellet, free of blood cells. These results are in agreement with the values for the content of the human genomic DNA in the two phases.

As this protocol uses relatively hard centrifugation conditions (20 minutes 1200 g) the g-forces and the centrifugation time was diminished in a first set of experiments to get a good recovery rate for the bacteria in the supernatant.

It was found that a 5 minute spin at 130 or 350 g (= 800 rpm or 1500 rpm) gave a recovery rate for *Staph. aureus* and P. *aeruginosa* in the range of about 80 to 90 % in the plasma phase.

There was no difference for the recovery rate between 130 g and 350 g, indicating that the bacteria are not able to penetrate significally into the dense Ficoll-like media.

Using these centrifugation conditions, the blood cell pellet in the Ficoll-like phase was more cloudy than compact but nevertheless the content of human genomic DNA in the plasma phase containing the bacteria was still in the range of about 1%.

It was furthermore possible to centrifuge the incubated sample without diluting it with PBS/RPLA-4, therefore avoiding the dilution of the initial content of bacteria in the supernatant.

**Conclusions**

Using a RosetteSep. antibody cocktail for leukocyte depletion, it was possible to coprecipitate the blood cells by a 20 minutes incubation step and to sediment the cells into a Ficoll-like media by a short centrifugation step (5 minutes 130 or 350 g). As the depletion of the leukocytes was very effective, it should even be possible to shorten the time of the incubation step. The recovery for *Staph. aureus* and P. *aeruginosa* in the plasma fraction was in the range of about 80 % to 90 %.

Therefore this protocol is able to deplete leukocytes from whole blood samples very effectively without loosing a significant amount of bacteria by providing antibodies specific for eukaryotic cells, whereby said antibodies are deficient of a bacteria-binding Fc-terminus.

### Example 4

**Isolation of bacteria from blood samples using magnetic beads and antibodies**

A more convenient protocol could be a format which combines antibodies deficient of a bacteria-binding Fc-terminus (for example the tetrameric antibody / immuno-precipitation approach described in example 3) with magnet bead technology separation. Such a format has the advantage that it could easily be integrated into an automated device such as for example the MagNA Pure®-system (Roche Diagnostics).

In such an approach the leukocytes would be co-precipitated via antibody eukaryotic cell complexes directly or indirectly bound to magnet beads (instead of erythrocytes as in the tetrameric antibody/ immuno-precipitation approach). In such an approach for example digoxigenin polyhapten coated beads and < Dig> antibodies could be used.

Undesired interactions between immunoglobulin binding proteins of the bacteria and the immunoreagent used seems to be excluded due to the blocking of the Fcγ-part of the IgG's present in the tetrameric antibody complexes.

## List of References

Åkerström, B., et al., J. Biol. Chem. 264 (1989) 19740-19746
Bakken, L.R., and Olsen, R.A., Appl. Environ. Microbiol. 45 (1983) 1188-1195
EP 0 320 308 B1
EP-0 329 822
Martineau, F., et al., J. Clin. Microbiol. 36 (1998) 618-623
Martinez-Salas, E., et al., J. Bacteriol. 147 (1981) 97—100
Navarre, W.W., and Schneewind, O., Microbiol. Mol. Biol. Rev. 63 (1999) 174-229
Nilson, B., et al., J. Immunol. Methods 99 (1987) 39-45
Rantakokko-Jalava, K., and Jalava, J., J. Clin. Microbiol. 40 (2002) 4211-4217
Reeves, H.C., et al., Anal. Biochem. 115 (1981) 194-196
Reischl, U., et al., J. Clin. Microbiol. 38 (2000) 2429-2433
Sambrook et al., Molecular Cloning - A Laboratory Manual, Cold Spring Harbor Laboratory Press (1989)
US 2003/0092078
US 4,786,600
US 4,876,187
US 5, 455,166
US 5,011,769
US 5,130,238
US 5,185,243
US 5,399,491
US 5,403,711
US 5,409,818
US 5,541,311
US 5,573,907
US 5,614, 402
US 5,660,988
US 5,679,524
US 5,705,365
US 5,710,029
US 5,719,028
US 5,843,669
US 5,846,717
US 5,888,779
US-A-4,683,202
Weinstein, M.P., et al., Clin. Infect. Dis. 24 (1997) 584-602
WO 89/09835
WO 89/12696
WO 90/01069
WO 95/00667
WO 95/05480
Woldringh, C.L., et al., J. Bacteriol. 148 (1981) 58-63

**Claims**

1. Method for isolation of bacteria from a biological sample comprising the steps:

    - providing antibodies specifically binding to eukaryotic cells contained in said biological sample, whereby said antibodies are deficient of a bacteria-binding Fc-terminus, and
    - mixing said antibodies and said biological sample, and
    - separating the antibody-eukaryotic cell-complexes from said mixture.

2. Method for extracting bacterial nucleic acids and/or bacterial proteins from a biological sample comprising the steps:

    - providing antibodies specifically binding to eukaryotic cells contained in said biological sample, whereby said antibodies are deficient of a bacteria-binding Fc-terminus, and
    - mixing said antibodies and said biological sample, and
    - separating the antibody-eucaryotic cell-complexes from said mixture, and
    - extracting bacterial nucleic acids and/or proteins contained in said mixture from which the antibody-eukaryotic cell-complexes are separated.

3. Method for detecting bacterial nucleic acids and/or bacterial proteins in a biological sample comprising the steps:

    - providing antibodies specifically binding to eukaryotic cells contained in said biological sample, whereby said antibodies are deficient of a bacteria-binding Fc-terminus, and
    - mixing said antibodies and said biological sample, and
    - separating the antibody-eucaryotic cell-complexes from said mixture, and
    - extracting bacterial nucleic acids and/or proteins contained in said mixture from which the antibody-eukaryotic cell-complexes are separated, and
    - detecting bacterial nucleic acids and/ or bacterial proteins contained in said biological sample.

4. Method of claim 3 for detecting bacterial nucleic acids in a biological sample, wherein said bacterial nucleic acids are detected by a nucleic acid amplification reaction optionally including a probe hybridization step.

5. Method according to claims 1 to 4, whereby said antibody-eukaryotic cell-complexes are separated from said biological sample by centrifugation.

6. Method according to claim 5, wherein centrifugation is performed in the presence of a density gradient media.

7. Method according to claims 1 to 4, wherein said antibody-eukaryotic cell-complexes are separated from said biological sample by filtration.

8. Method according to claims 1 to 4, wherein said antibodies are directly or indirectly coated on magnetic beads.

9. Method of any of claims 1 to 8, wherein said antibodies are Tetrameric Antibody Complexes.

10. Method of any of claims 1 to 8, wherein said antibodies are Fab-fragments or F(ab')2-fragments.

11. Method of any of claims 1 to 8, wherein said antibodies are masked at the Fc-termini.

12. Method of any of claims 1 to 8, wherein said antibodies are Ig-M-type antibodies.

13. Use of antibodies specifically binding to eukaryotic cells which are deficient of a bacteria-binding Fc-terminus for depletion of eukaryotic cells in a biological sample in a method for isolation of bacteria from a biological sample.

14. Use of antibodies specifically binding to eukaryotic cells which are deficient of a bacteria-binding Fc-terminus for depletion of eukaryotic cells in a biological sample in a method for extracting bacterial nucleic acids and/ or proteins from a biological sample.

15. Use of antibodies specifically binding to eukaryotic cells which are deficient of a bacteria-binding Fc-terminus for depletion of eukaryotic cells in a biological sample in a method for detecting bacterial nucleic acids and/ or proteins in a biological sample.

16. Kit for extracting bacterial nucleic acids and/ or bacterial proteins from a biological sample comprising:

- in one or several containers antibodies specifically binding to eukaryotic cells contained in said biological sample, whereby said antibodies are deficient of a bacteria-binding Fc-terminus, and
- in one or several containers means for extracting nucleic acids and/ or proteins.

17. Kit for detecting bacterial nucleic acids and/ or bacterial proteins in a biological sample further comprising:

- in one or several containers antibodies specifically binding to eukaryotic cells contained in said biological sample, whereby said antibodies are deficient of a bacteria-binding Fc-terminus, and
- in one or several containers means for extracting nucleic acids and/ or proteins, and
- means for detecting nucleic acids and/ or proteins.

18. Kit for detecting bacterial nucleic acids in a biological sample further comprising:

- in one or several containers antibodies specifically binding to eukaryotic cells contained in said biological sample, whereby said antibodies are deficient of a bacteria-binding Fc-terminus, and
- in one or several containers means for extracting nucleic acids and/ or proteins, and
- means for detecting nucleic acids and/ or proteins, and
- means for amplifying a bacterial nucleic acid target region, preferably by PCR.

## Patentansprüche

1. Verfahren zur Isolierung von Bakterien aus einer biologischen Probe, bei dem man in den folgenden Schritten

- spezifisch an in der biologischen Probe enthaltene eukaryontische Zellen bindende Antikörper bereitstellt, wodurch den Antikörpern ein bakterienbindender Fc-Terminus fehlt, und
- die Antikörper und die biologische Probe mischt und
- die Komplexe aus Antikörper und eukaryontischer Zelle von dem Gemisch trennt.

2. Verfahren zur Extraktion bakterieller Nukleinsäuren und/oder bakterieller Proteine aus einer biologischen Probe, bei dem man in den folgenden Schritten

- spezifisch an in der biologischen Probe enthaltene eukaryontische Zellen bindende Antikörper bereitstellt, wodurch den Antikörpern ein bakterienbindender Fc-Terminus fehlt, und
- die Antikörper und die biologische Probe mischt und
- die Komplexe aus Antikörper und eukaryontischer Zelle von dem Gemisch trennt und
- in dem Gemisch, von dem die Komplexe aus Antikörper und eukaryontischer Zelle getrennt werden, enthaltene bakterielle Nukleinsäuren und/oder Proteine extrahiert.

3. Verfahren zum Nachweis bakterieller Nukleinsäuren und/oder bakterieller Proteine in einer biologischen Probe, bei dem man in den folgenden Schritten

- spezifisch an in der biologischen Probe enthaltene eukaryontische Zellen bindende Antikörper bereitstellt, wodurch den Antikörpern ein bakterienbindender Fc-Terminus fehlt, und
- die Antikörper und die biologische Probe mischt und
- die Komplexe aus Antikörper und eukaryontischer Zelle von dem Gemisch trennt und
- in dem Gemisch, von dem die Komplexe aus Antikörper und eukaryontischer Zelle getrennt werden, enthaltene bakterielle Nukleinsäuren und/oder Proteine extrahiert und
- in der biologischen Probe enthaltene bakterielle Nukleinsäuren und/oder bakterielle Proteine nachweist.

4. Verfahren nach Anspruch 3 zum Nachweis von bakteriellen Nukleinsäuren in einer biologischen Probe, wobei die bakteriellen Nukleinsäuren mit einer Nukleinsäureamplifikationsreaktion, die gegebenenfalls einen Sondenhybridisierungsschritt beinhaltet, nachgewiesen werden.

5. Verfahren gemäß Ansprüchen 1 bis 4, wodurch die Komplexe aus Antikörper und eukaryontischer Zelle von der biologischen Probe mittels Zentrifugation getrennt werden.

6. Verfahren gemäß Anspruch 5, wobei die Zentrifugation in Gegenwart eines Dichtegradientenmediums durchgeführt wird.

7. Verfahren gemäß Ansprüchen 1 bis 4, wobei die Komplexe aus Antikörper und eukaryontischer Zelle von der biologischen Probe mittels Filtration getrennt werden.

8. Verfahren gemäß Ansprüchen 1 bis 4, wobei magnetische Kügelchen direkt oder indirekt mit den Antikörpern beschichtet werden.

9. Verfahren nach einem der Ansprüche 1 bis 8, wobei es sich bei den Antikörpern um "Tetrameric Antibody Complexes" [tetramere Antikörperkomplexe] handelt.

10. Verfahren nach einem der Ansprüche 1 bis 8, wobei es sich bei den Antikörpern um Fab-Fragmente oder F(ab')2-Fragmente handelt.

11. Verfahren nach einem der Ansprüche 1 bis 8, wobei die Antikörper an den Fc-Termini maskiert sind.

12. Verfahren nach einem der Ansprüche 1 bis 8, wobei es sich bei den Antikörpern um Antikörper vom Typ Ig M handelt.

13. Verwendung von spezifisch an eukaryontische Zellen bindenden Antikörpern, denen ein bakterienbindender Fc-Terminus fehlt, zur Abreicherung eukaryontischer Zellen in einer biologischen Probe in einem Verfahren zur Isolierung von Bakterien aus einer biologischen Probe.

14. Verwendung von spezifisch an eukaryontische Zellen bindenden Antikörpern, denen ein bakterienbindender Fc-Terminus fehlt, zur Abreicherung eukaryontischer Zellen in einer biologischen Probe in einem Verfahren zur Extraktion bakterieller Nukleinsäuren und/oder Proteinen aus einer biologischen Probe.

15. Verwendung von spezifisch an eukaryontische Zellen bindenden Antikörpern, denen ein bakterienbindender Fc-Terminus fehlt, zur Abreicherung eukaryontischer Zellen in einer biologischen Probe in einem Verfahren zum Nachweis bakterieller Nukleinsäuren und/oder Proteinen in einer biologischen Probe.

16. Kit zur Extraktion bakterieller Nukleinsäuren und/oder bakterieller Proteine aus einer biologischen Probe, umfassend:

- in einem oder mehreren Behältern Antikörper, die spezifisch an in der biologischen Probe enthaltene eukaryontische Zellen binden, wodurch den Antikörpern ein bakterienbindender Fc-Terminus fehlt, und
- in einem oder mehreren Behältern Mittel zur Extraktion von Nukleinsäuren und/oder Proteinen.

17. Kit zum Nachweis bakterieller Nukleinsäuren und/oder bakterieller Proteine in einer biologischen Probe, ferner umfassend:

- in einem oder mehreren Behältern Antikörper, die spezifisch an in der biologischen Probe enthaltene eukaryontische Zellen binden, wodurch den Antikörpern ein bakterienbindender Fc-Terminus fehlt, und
- in einem oder mehreren Behältern Mittel zur Extraktion von Nukleinsäuren und/oder Proteinen sowie
- Mittel zum Nachweis von Nukleinsäuren und/oder Proteinen.

18. Kit zum Nachweis bakterieller Nukleinsäuren in einer biologischen Probe, ferner umfassend:

- in einem oder mehreren Behältern Antikörper, die spezifisch an in der biologischen Probe enthaltene eukaryontische Zellen binden, wodurch den Antikörpern ein bakterienbindender Fc-Terminus fehlt, und
- in einem oder mehreren Behältern Mittel zur Extraktion von Nukleinsäuren und/oder Proteinen und
- Mittel zum Nachweis von Nukleinsäuren und/oder Proteinen sowie
- Mittel zur Amplifikation eines Zielbereichs einer bakteriellen Nukleinsäure, vorzugsweise durch PCR.

**Revendications**

1. Procédé pour l'isolation de bactéries à partir d'un échantillon biologique, comprenant les étapes :

   - procurer des anticorps se liant de manière spécifique à des cellules eucaryotes que contient ledit échantillon biologique, lesdits anticorps manifestant une déficience quant au fragment Fc terminal de liaison à des bactéries ; et
   - mélanger lesdits anticorps et ledit échantillon biologique ; et
   - séparer dudit mélange les complexes anticorps-cellules eucaryotes.

2. Procédé pour l'extraction d'acides nucléiques bactériens et/ou de protéines bactériennes à partir d'un échantillon biologique, comprenant les étapes :

   - procurer des anticorps se liant de manière spécifique à des cellules eucaryotes que contient ledit échantillon biologique, lesdits anticorps manifestant une déficience quant au fragment Fc terminal de liaison à des bactéries ; et
   - mélanger lesdits anticorps et ledit échantillon biologique ; et
   - séparer dudit mélange les complexes anticorps-cellules eucaryotes ; et
   - extraire les acides nucléiques bactériens et/ou les protéines bactériennes que contient ledit mélange dont les complexes anticorps-cellules eucaryotes ont été séparés.

3. Procédé pour la détection d'acides nucléiques bactériens et/ou de protéines bactériennes dans un échantillon biologique, comprenant les étapes :

   - procurer des anticorps se liant de manière spécifique à des cellules eucaryotes que contient ledit échantillon biologique, lesdits anticorps manifestant une déficience quant au fragment Fc terminal de liaison à des bactéries ; et
   - mélanger lesdits anticorps et ledit échantillon biologique ; et
   - séparer dudit mélange les complexes anticorps-cellules eucaryotes ; et
   - extraire les acides nucléiques bactériens et/ou les protéines bactériennes que contient ledit mélange dont les complexes anticorps-cellules eucaryotes ont été séparés ; et
   - détecter les acides nucléiques bactériens et/ou les protéines bactériennes que contient ledit échantillon biologique.

4. Procédé selon la revendication 3 pour la détection d'acides nucléiques bactériens dans un échantillon biologique, dans lequel lesdits acides nucléiques bactériens sont détectés via une réaction d'amplification d'acides nucléiques englobant facultativement une étape d'hybridation par sonde.

5. Procédé selon les revendications 1 à 4, par lequel lesdits complexes anticorps-cellules eucaryotes sont séparés dudit échantillon biologique par centrifugation.

6. Procédé selon la revendication 5, dans lequel la centrifugation est mise en oeuvre en présence d'un milieu à gradient de densité.

7. Procédé selon les revendications 1 à 4, dans lequel lesdits complexes anticorps-cellules eucaryotes sont séparés dudit échantillon biologique par filtration.

8. Procédé selon les revendications 1 à 4, dans lequel lesdits anticorps enrobent directement ou indirectement des billes magnétiques.

9. Procédé selon l'une quelconque des revendications 1 à 8, dans lequel lesdits anticorps sont des complexes d'anticorps tétramères.

10. Procédé selon l'une quelconque des revendications 1 à 8, dans lequel lesdits anticorps sont des fragments Fab ou des fragments F(ab')2.

11. Procédé selon l'une quelconque des revendications 1 à 8, dans lequel lesdits anticorps sont masqués aux fragments Fc terminaux.

**12.** Procédé selon l'une quelconque des revendications 1 à 8, dans lequel lesdits anticorps sont des anticorps de type Ig-M.

**13.** Utilisation d'anticorps se liant de manière spécifique à des cellules eucaryotes, qui manifestent une déficience quant au fragment Fc terminal de liaison à des bactéries à des fins de déplétion des cellules eucaryotes dans un échantillon biologique dans un procédé pour isoler des bactéries d'un échantillon biologique.

**14.** Utilisation d'anticorps se liant de manière spécifique à des cellules eucaryotes, qui manifestent une déficience quant au fragment Fc terminal de liaison à des bactéries à des fins de déplétion des cellules eucaryotes dans un échantillon biologique dans un procédé pour extraire des acides nucléiques bactériens et/ou des protéines bactériennes à partir d'un échantillon biologique.

**15.** Utilisation d'anticorps se liant de manière spécifique à des cellules eucaryotes, qui manifestent une déficience quant au fragment Fc terminal de liaison à des bactéries à des fins de déplétion des cellules eucaryotes dans un échantillon biologique dans un procédé pour détecter des acides nucléiques bactériens et/ou des protéines bactériennes dans un échantillon biologique.

**16.** Nécessaire pour l'extraction d'acides nucléiques bactériens et/ou de protéines bactériennes à partir d'un échantillon biologique, comprenant :

- dans un ou plusieurs contenants, des anticorps se liant de manière spécifique à des cellules eucaryotes que contient ledit échantillon biologique, lesdits anticorps manifestant une déficience quant à un fragment Fc terminal de liaison à des bactéries ; et
- dans un ou plusieurs contenants, des moyens pour extraire des acides nucléiques et/ou des protéines.

**17.** Nécessaire pour la détection d'acides nucléiques bactériens et/ou de protéines bactériennes dans un échantillon biologique, comprenant en outre :

- dans un ou plusieurs contenants, des anticorps se liant de manière spécifique à des cellules eucaryotes que contient ledit échantillon biologique, lesdits anticorps manifestant une déficience quant à un fragment Fc terminal de liaison à des bactéries ; et
- dans un ou plusieurs contenants, des moyens pour extraire des acides nucléiques et/ou des protéines ; et
- des moyens pour détecter des acides nucléiques et/ou des protéines.

**18.** Nécessaire pour la détection d'acides nucléiques bactériens dans un échantillon biologique, comprenant en outre :

- dans un ou plusieurs contenants, des anticorps se liant de manière spécifique à des cellules eucaryotes que contient ledit échantillon biologique, lesdits anticorps manifestant une déficience quant à un fragment Fc terminal de liaison à des bactéries ; et
- dans un ou plusieurs contenants, des moyens pour extraire des acides nucléiques et/ou des protéines ; et
- des moyens pour détecter des acides nucléiques et/ou des protéines ; et
- des moyens pour amplifier une zone cible d'acide nucléique bactérien, de préférence par PCR.